# EUROPEAN PATENT SPECIFICATION

(11) **EP 2 048 246 B1**
(45) Date of publication and mention of the grant of the patent: **14.09.2011**
(21) Application number: 07790559.4
(22) Date of filing: 10.07.2007
(51) Int. Cl.: C12Q 1/68, C12N 15/09

(54) **METHOD FOR OBTAINING OLIGONUCLEOTIDE**
VERFAHREN ZUR GEWINNUNG EINES OLIGONUKLEOTIDS
PROCÉDÉ DE PRÉPARATION D'UN OLIGONUCLÉOTIDE

(30) Priority: 03.08.2006 JP 2006212046
(43) Date of publication of application: 15.04.2009
(73) Proprietor: NEC Soft, Ltd., Tokyo 136-0082 (JP); National Institute of Advanced Industrial Science and Technology, Tokyo 100-8921 (JP)
(72) Inventor: NISHIKAWA, Fumiko, Tsukuba-shi, Ibaraki 305-8566 (JP); NISHIKAWA, Satoshi, Tsukuba-shi, Ibaraki 305-8566 (JP); FURUICHI, Makio, Tokyo 136-0082 (JP); MIZUNO, Hiroshi, Tokyo 136-0082 (JP); WAGA, Iwao, Tokyo 136-0082 (JP)
(74) Representative: Betten & Resch
(86) International application number: PCT/JP2007/063741
(87) International publication number: WO 2008/015884

(56) References cited:
- WO-A-03/102212
- WO-A2-03/102212
- US-A- 5 707 796
- US-B1- 6 465 189
- CONRAD R C ET AL: "IN VITRO SELECTION OF NUCLEIC ACID APTAMERS THAT BIND PROTEINS" METHODS IN ENZYMOLOGY, ACADEMIC PRESS INC, SAN DIEGO, CA, US, vol. 267, 1 January 1996 (1996-01-01), pages 336-367, XP000999597 ISSN: 0076-6879
- MENDONSA S D ET AL: "In vitro selection of high-affinity DNA ligands for human IgE using capillary electrophoresis" ANALYTICAL CHEMISTRY, AMERICAN CHEMICAL SOCIETY. COLUMBUS, US, vol. 76, no. 18, 1 January 2004 (2004-01-01), pages 5387-5392, XP003020887 ISSN: 0003-2700
- MOSING R K ET AL: "Capillary electrophoresis-SELEX selection of aptamers with affinity for HIV-1 reverse transcriptase" ANALYTICAL CHEMISTRY, AMERICAN CHEMICAL SOCIETY. COLUMBUS, US, vol. 77, no. 19, 1 January 2005 (2005-01-01), pages 6107-6112, XP003020888 ISSN: 0003-2700
- CONRAD R.C. ET AL.: 'In vitro selection of nucleic acid aptamers that bind proteins' METHODS ENZYMOL. vol. 267, 1996, pages 336 - 367, XP000999597
- LI Y. ET AL.: 'A modified quantitative EMSA and its application in the study of RNA-protein interactions' J. BIOCHEM. BIOPHYS. METHODS vol. 60, 2004, pages 85 - 96, XP003020885
- RODGERS J.T. ET AL.: 'Use of biotin-labeled nucleic acids for protein purification and agarose-based chemiluminescent electromobility shift assays' ANAL. BIOCHEM. vol. 277, 2000, pages 254 - 259, XP003020886
- MENDONSA S.D. ET AL.: 'In vitro selection of high-affinity DNA ligands for human IgE using capillary electrophoresis' ANAL. CHEM. vol. 76, no. 18, 2004, pages 5387 - 5392, XP003020887
- MOSING R.K. ET AL.: 'Capillary electrophoresis-SELEX selection of aptamers with affinity for HIV-1 reverse transcriptase' ANAL. CHEM. vol. 77, no. 19, 2005, pages 6107 - 6112, XP003020888
- MARSHALL K.A. ET AL.: 'In vitro selection of RNA aptamers' METHODS ENZYMOL. vol. 318, 2000, pages 193 - 214, XP001031309
- BEREZOVSKI M. ET AL.: 'Non-SELEX selection of aptamers' J. AM. CHEM. SOC. vol. 128, January 2006, pages 1410 - 1411, XP003020889

## Description

### Field of Invention

The present invention relates to a method for obtaining oligonucleotide.

### Related Art

Although it has believed that oligonucleotides such as DNA and RNA mainly have a function, as molecules concerning the synthesis of proteins, it has numerously found that molecules perform the expression of function thereof by means of the interaction with gene, e.g. ribozyme and RNai. Attention is currently focused on the application for therapeutical field. Especially, the aptamer has been paid attention as a nucleic acid which binds to macromolecule such as proteins capable of altering the function of the macromolecule. In order to apply it to medicines, new aptamers have been obtained.

The aptamer such as RNA aptamer has been generally obtained in accordance with SELEX method (Systematic Evolution of Ligands by Exponential enrichment) (see Patent Document 1). In case of obtaining RNA aptamer with SELEX method, a target substance such as protein is associated with DNA library comprising randomized sequence to form a complex of RNA/target substance. Next, the complex of RNA/target substance is attached on a protein-adsorptive filter such as nitrocellulose filter. Free RNA which is not involved to form the complex is included on the filter, in addition to the complex or RNA/target substance. The free RNA is physically washed from the filter by using a buffer. The filter is treated with a buffer containing any agent including urea to obtain a desired RNA from the complex of RNA/target substance which is included on the filter. The obtained RNA is amplified with PCR methods to obtain a gene product, and the above-mentioned steps of the formation of complex, and the wishing and treatment through the filter are repeated. The SELEX method is a method for obtaining gene products which can specifically bind to target substance by means of repeating such steps.

In the method for obtaining the oligonucleotide such as RNA aptamer in accordance with the SELEX method, it is necessary to attach a mixture containing the complex of oligonucleotide/target substance which is obtained to associate an oligonucleotide library such as DNA library with the target substance, to the protein-adsorptive filter such as nitrocellulose filter. This will result in binding the complex of oligonucleotide/target substance on the filter. In addition, the oligonucleotide which cannot be bound to the target substance, that is, the oligonucleotide not to be subjected to obtain with this method will be also attached on the filter by means of nonspecific adsorption it with the filter. Accordingly, in order to only obtain the desired oligonucleotide from the complex of oligortucleotide/target substance, it is necessary to further perform steps of washing the filter so as not to remain an undesired oligonucleotide on the filter and to remain the complex of oligonucleotide/target substance on the filter and of extracting the desired oligonucleotide from the filter. Therefore, it is difficult to efficiently obtain the desired oligonucleotide by means of the above-mentioned washing step and extracting step due not only to the interaction of the undesired oligonucleotide with the filter but also to the interaction of the undesired oligonucleotide with the complex of oligonucleotide/target substance.

In addition, it is necessary to repeat each step as mentioned above with several to ten or more times in the SELEX method in order to obtain the oligonucleotide having high binding capacity to the target substance. So, it has a disadvantage in view of cost and operating efficiency.

Further, it is necessary to use an apparatus including the filter manifold for the above-mentioned washing/extracting step. The method is unsuitable for obtaining the oligonucleotide with large scale.

### Patent Document 1

JP 2763958B
The document by CONRAD R.C. ET AL.: "IN VITRO SELECTION OF NUCLEIC ACID APTAMERS THAT BIND PROTEINS", METHODS IN ENZYMOLOGY, Vol. 267, 1 January 1996 (1996-01-01), pages 336-367, discloses a method for obtaining a RNA molecule with a high binding capacity to a protein, whereby a combination of modified cellulose filters and polyacrylamide gel electrophoresis is used to separate the RNA/ protein complex from unbound RNA molecules. Afterwards, the RNA/protein complex is eluted, the target RNA molecule is recovered and reverse transcribed for PCR amplification. Amplified ds-DNA molecules are then transcribed to provide the desired amount of RNA target molecules.
Document "A MODIFIED QUANTITATIVE EMSA AND ITS APLLICATION", J. BIOCHEM. BIOPHYS. METHODS, Vol. 60, 2004, Pages 85-96, by Li Y. et al., discloses agarose gel electrophoesis for separating a RNA/protein complex.

### Disclosure of the invention

### Problems to be solved

The present invention is based on such a problem, and is to provide a method for obtaining an oligonucleotide such as RNA aptamer having high binding capacity to a target substance with easy-to-use and high specificity.

### Means to solve the problems

The method for obtaining oligonucleotide according to the present invention is defined in claim 1.
Advantageous embodiments are defined in the dependent claims.

### Effect of Invention

In accordance with the present invention, it is possible to obtain the oligonucleotide having high binding capacity with an advantage in cost and in a short time.

In addition, it is possible to obtain such an oligonucleotide in bulk with small scale, which is impossible in the prior art.

### Brief explanation of Drawing

Figure 1 is an electrophoresis image as obtained in the Reference Example 1.
Figure 2 is an electrophoresis image as obtained in the Reference Example 2.
Figure 3 is a visualized image as obtained in the Embodiment 2 and the Comparative Examples 1 and 2.
Figure 4 is an autoradiogram as obtained in the Embodiment 2-1 at second generation.
Figure 5 is an autoradiogram as obtained in the Embodiment 2-1 at third generation.
Figure 6 is an autoradiogram as obtained in the Embodiment 2-1 at fourth generation.

### Best mode for carrying out the invention

### <The method for obtaining oligonucleotide according to the present invention>

The method for obtaining oligonucleotide according to the present invention is defined in claim 1. Hereinafter, the "step of performing an electrophoresis of a nucleic acid molecule/target substance complex comprising a nucleic acid molecule, and a target substance" is referred to as an electrophoresis step, the "step of recovering said nucleic acid molecule/target substance complex" is referred to as a recovery step, the "step of extracting the nucleic acid molecule from said nucleic acid molecule/target substance complex" is referred to as an extraction step and the "step of gene amplifying said nucleic acid molecule." is referred to as an amplification step.

The method for obtaining oligonucleotide according to the present invention is a method for obtaining the oligonucleotide which can be bound to the target substance, from the nucleic acid molecule wherein it performs the steps of the electrophoresis step, the recovery step, the extraction step and the amplification step to obtain the gene product such as DNA and RNA, and if necessary, the obtained gene product is subjected to perform the steps including the electrophoresis step and the following steps.

### (The electrophoresis step)

The electrophoresis step in the method for obtaining oligonucleotide according to the present invention is performed such that the mixture containing nucleic acid molecule such as single strand RNA and nucleic acid molecule/target substance complex comprising the target substance such as proteins is introduced into an appropriate substrate such as agarose gel and a voltage is applied to the substrate. This will result in separating the nucleic acid molecule and the nucleic acid molecule/target substance complex contained in the mixture due to the difference in mobility in the substrate.

In the method for obtaining oligonucleotide according to the present invention, the electrophoresis step is performed with at least 1 to 3 times, specially, preferably with once in the agarose gel in view of efficiently obtaining the oligonucleotide. In addition, in the method for obtaining oligonucleotide according to the present invention, the agarose gel is preferably used for the first time of the electrophoresis step. This will enable to efficiently remove the nucleic acid molecule such as DNA molecule which non-specifically binds. Further, in the method for obtaining oligonucleotide according to the present invention, the method preferably comprises the electrophoresis step using the agarose gel and the electrophoresis step using the polyacrylamide gel. This will enable to fractionate and obtain candidates to be subjected such as aptamer with easy-to-handle and in a short time. Such a candidate can be specifically fractionated and obtained in the polyacrylamide gel method.

### [The nucleic acid molecule]

In the present invention, the nucleic acid molecule generically refers to as a so-called nucleic acid including adenine, guanine, cytosine, thymine and uracil, and/or so-called gene-related substances of nucleoside comprising nucleic acid analog substance in which the sugar portion of the nucleoside is ester-linked with the phosphate residue thereof. For example, example of the nucleic acid molecule includes single strand DNA, single strand DNA, double strand DNA and double strand RNA. Hereinafter, it will be described with regard to the single strand RNA (RNA aptamer) to be subjected to obtain.

In this case, the nucleic acid molecule may comprise: a region containing randomized sequence consisting of A, G, C and U (hereinafter, referred also to as a random region); and a fixed sequence containing a known sequence at 5'-end and 3'-end of the random region (hereinafter, referred also to as a 5'-end fixed sequence and a 3'-send fixed sequence, respectively). These fixed sequences correspond to a primer region necessary for PCR methods for performing the amplification step as mentioned below in the method for obtaining oligonucleotide according to the present invention.

Such a nucleic acid molecule can be easily obtained as the following method. That is, a gene product comprising a complementary DNA to the above-mentioned single strand RNA ; (5'-end fixed sequence-random region-3' -end fixed sequence) may be preliminary prepared, and the object nucleic acid molecule may be prepared from such a gene product with DNA-dependent RNA polymerase such as T7 RNA polymerase in accordance with in vitro transcription method. In this case, a sequence capable of activating the promoter activity of the DNA dependent RNA polymerase as used may be linked to the upstream of the complementary sequence to the 5'-end fixed sequence.

The 5'-end fixed sequence and 3'-send fixed sequence of the nucleic acid molecule is not limited so far as it has less effect for binding the nucleic acid molecule and target substance and it provides a primer region necessary for PCR methods in the amplification step. In case of using single strand RNA as the nucleic acid molecule, the 3'-end fixed sequence will offer the primer region for RNA dependent DNA polymerase and DNA dependent DNA polymerase in the amplification steep, and the 5'-end fixed sequence will offer the primer region for DNA dependent DNA polymerase in the amplification step. Accordingly, the 5'-end fixed sequence and 3'- end fixed sequence may be appropriately selected based on the expression profile of the activity of these polymerases.

The random region of the nucleic acid molecule is a gene comprising A, G, C and U (in the sequence listing, correctively referred to as "n") having a predetermined base length. The base length may be appropriately altered based on the oligonucleotide to be subjected to obtain and on the property of the target substance, and may be, for example, 15 to 60 bases.

A method for manufacture of the random region is not limited, including artificial synthesis, semi-artificial synthesis. For example, example of such a method includes a synthesis method using nucleic acid synthesis apparatuses such at 3400 DNA synthesizer (Applied Biosystems), an extraction method according to the well known method such as the phenol extraction of natural origin gene products, and a preparation method of cleaving such a gene product with gene cleavage enzymes.

It should be noted that the present invention is not limited to use the single strand RNA, although the above-mentioned description is for the single strand RNA as one of examples of the nucleic acid molecule used in the method for obtaining oligonucleotide according to the present invention. With regard to the other molecular species, the nucleic acid molecule can be prepared by appropriately selecting the sequence, primer and enzymes and the others based on the molecular species.

### [The target substance]

The target substance means a counterpart substance to be obtained in the present invention. Example of the target substance includes molecules, compounds and substances which can form a complex with nucleic acid molecule by means of the intermolecular associations such as hydrogen bonding and ion binding with the oligonucleotide. For example, example of the target substance includes peptides, proteins, glycoproteins, hormones, growth factors, receptors, antigens, antibodies, nucleic acids, polysaccharides, carbohydrates and synthetic compounds.

### [The nucleic acid molecule/target substance complex]

The nucleic acid molecule/target substance complex comprising the nucleic acid molecule and the target substance is prepared by incubating the nucleic acid molecule and the target substance in buffers such as Tris-based or in an appropriate solvent for a predetermined period. In this case, for the purpose of efficiency of the electrophoresis step, high density solution such a glycerol is preferably added. Example of such an additive includes glycerol and saccharose solution. The amount of the additive is not limited so far as the formation of the nucleic acid molecule/target substance complex is not inhibited. According to the invention, the amount is in the range of 10 to 30 w/v% (10 to 30 v/v% with regard to the volume of the reaction mixture). In addition, for the purpose of facilitating the formation of the nucleic acid molecule/target substance complex, monovalent or divalent cationic ion may be added.

A method for preparing the nucleic acid molecule/target substance complex, but not limited to, may be such that the nucleic acid molecule and the target substance is added to and in contact with the above-mentioned appropriate solvent. Such a contact is performed at which the substrate is introduced, in case of performing the electrophoresis step. In addition, it may be performed in an appropriate container.

### [The substrate]

In the present invention, the substrate used in the electrophoresis step is not limited to so far as the nucleic acid molecule and the nucleic acid molecule/target substance complex contained in the mixture can be electrically separated. Example of the substrate includes agarose gel, polyacrylamide gel and watersoluble cellulose gel. The concentration of the substrate is not limited to so far as the nucleic acid molecule and the nucleic acid molecule/target substance complex can be separated in the substrate. For example, in case of using agarose as the substrate, the concentration of the substrate may be in the range of 0.5% to 2.0%, preferably 0.7% to 1.5%. In addition, in case of using polyacrylamide gel as the substrate, the concentration of the substrate may be in the range of 5 to 20%, preferably 7 to 12%.

In the electrophoresis step, the substrate may be prepared to solve in buffers capable of electrically charging the nucleic acid molecule, the nucleic acid molecule/target substance complex and the others. A well-known buffer which can be used for the electrophoresis may be used, including Tris-based and HEPES-based buffer. Among them, in case of performing the electrophoresis step using agarose gel, example of the buffer includes TBE buffer (Tris-borate-EDTA buffer) and TAE buffer (Tris-acetate-EDTA buffer).

The electrophoresis in the electrophoresis step may be performed such that the binding condition of the nucleic acid molecule/target substance complex is maintained in the substrate and an appropriate voltage is applied for the mixture introduced in the substrate so as to be capable of transferring the nucleic acid molecule and the nucleic acid molecule/target substance complex in the substrate with different mobility. The range of the voltage and the applied time may be appropriately selected in view of the mobility of the mixture in the substrate. For example, in case of performing the electrophoresis step using the agarose gel, the electrophoresis can be performed for 5 to 30 minutes at the voltage of 50 to 150V. In case of performing the electrophoresis step using the polyacrylamide gel, the electrophoresis can be performed for 1 to 4 hours at the voltage of 100 to 200 V. The applied time may be that the nucleic acid molecule/target substance complex and the nucleic acid molecule or the target substance which is not involved in the formation of the nucleic acid molecule/target substance complex can be electrophoretically separated. In addition, the temperature of the electrophoresis step is not limited so far as the mobile phase is not solidified, and may be the range of 2°C to room temperature.

The mobile phase performing the electrophoresis step may be used so far as the binding condition of the nucleic acid molecule/target substance complex is at least maintained, including a solvent containing a well-known buffer such as Tris. If necessary, electrolytes such as cationic ion and anionic ion may be added in the mobile phase. The mobile phase may contain monovalent or divalent cationic ion in view of maintaining the binding condition of the nucleic acid molecule/target substance complex.

So separated nucleic acid molecule/target substance complex from the nucleic acid molecule in the substrate is provided in the following recovery step.

### (The recovery step)

In the method for obtaining oligonucleotide according to the present invention, the recovery step is a step of recovering the nucleic acid molecule/target substance complex from the substrate as obtained in the electrophoresis step. The recovery step may be appropriately selected in accordance with the existence form of the nucleic acid molecule/target substance complex in the substrate. For example, example of means for carrying out the recovery step includes a method of directly inserting an appropriate dispensing apparatus or instrument such as pipette into the well of the substrate to collect the subject, or a method of cutting the substrate containing nucleic acid molecule/target substance complex with cutter to recover the subject as a piece of the substrate.

The nucleic acid molecule/target substance complex contained in the substrate after the electrophoresis step may be located in and near the well in which the mixture is introduced in the substrate, or may form the band shape in the substrate, depending on the condition of the electrophoresis.

In case of locating the nucleic acid molecule/target substance complex in the well, using the appropriate dispensing means such as pipette, the means may be inserted in the well to directly collect the nucleic acid molecule/target substance complex. This technique may be applied in case of locating the nucleic acid molecule/target substance complex near the well. In this case, the nucleic acid molecule/target substance complex will be recovered in the solution state.

Even in case of locating the nucleic acid molecule/target substance complex near the well, when the above-mentioned technique is difficult to use, the substrate near the well may be cut into the piece of the substrate having an appropriate size to collect the nucleic acid molecule/target substance complex along with the substrate. This technique can be applied in case that the nucleic acid molecule/target substance complex forms the band shape in the substrate. That is, in case of locating the nucleic acid molecule/target substance complex in the substrate, a part of the substrate containing the nucleic acid molecule/target substance complex may be cut with cutter to recover the nucleic acid molecule/target substance complex along with the substrate.

In the recovery step, one of the above-mentioned techniques or an appropriate combination thereof may be selected.

In the recovery step, depending on the property of the nucleic acid molecule and/or target substance to be subjected, an appropriate method capable of observing these molecules and/or substances may be appropriately used as a method for confirming the existence of the nucleic acid molecule/target substance complex in the substrate.

For example, example of the method of confirming the existence of the nucleic acid molecule in the substrate includes a method of visualizing the molecule using a well-known detecting agent for the gene product such as ethidium bromide and cybergreen. In addition, it may be that the nucleic acid molecule is preliminary labeled with radioactive compounds such as ³²P-ATP or with fluorescence agent such as fluorescein and Cy5, and these labels are detected.

In addition, in case of using protein as the target substance, example of the method of confirming the existence of the target substance in the substrate includes a method for visualizing the protein such as silver staining, coomassie brilliant blue (CBB) straining.

So recovered nucleic acid molecule/target substance complex will be provided in the following extraction step.

### (The extraction step)

In the method for obtaining oligonucleotide according to the present invention, the extraction step is a step of extracting the nucleic acid molecule from the nucleic acid molecule/target substance complex recovered in the recovery step. The extraction step may be appropriately performed in accordance with the existence form (e.g. the solution state, and a state along with the substrate) of the nucleic acid molecule/target substance complex obtained in the recovery step. Example of the recovery step includes a well-known method of recovering gene products such as phenol extraction, ethanol precipitation and spin column method.

In case that the nucleic acid molecule/target substance complex has been recovered in the solultion state, the nucleic acid molecule may be extracted from the nucleic acid molecule/target substance complex using the phenol extraction and ethanol precipitation and the others. In case that the nucleic acid molecule/target substance complex has been recovered along with the substrate, the nucleic acid molecule may be extracted from the nucleic acid molecule/target substance complex using the spin column having an appropriate cut off value based on the molecular weight and other property of the target substance.

So extracted nucleic acid molecule will be provided in the following amplification step.

### (The amplification step)

In the method for obtaining oligonucleotide according to the present invention, the amplification step is a step of amplifying the nucleic acid molecule obtained in the extraction step and the other steps. The amplification step is a step of the gene amplification using PCR methods such as RT-PCR, PCR and in vitro transcription. The amplification step may be performed, by utilizing the fixed sequence contained in the 5'-end and 3'-end of the nucleic acid molecule, with the primer which complementary binding to these fixed sequences. As an example, the amplification step using the single strand RNA will be explained.

First, the single strand DNA obtained in the extraction step, RNA dependent DNA polymerase (reverse transcriptase) such as reverse transcriptase originated from avian myeloblastosis virus (AMV Reverse Transcriptase), and the primer complementary to the fixed sequence at 3'-end of the single strand RNA are incubated to amplify the complementary DNA to this single strand RNA. So amplified complementary DNA are further amplified into a double strand DNA by using primers complementary to the 5'-end fixed sequence and the 3'-end fixed sequence contained in the complementary DNA and a well-known DNA dependent RNA polymerase such as Taq DNA polymerase. Then, the double strand DNA is transcribed into the single strand RNA with a well-known DNA dependent. RNA polymerase such as T7 RNA polymerase.

In this way, the subject nucleic acid molecule capable of binding to the target substance are specifically amplified through the steps of the electrophoresis step, the recovery step, the extraction step and the amplification step.

Although the single strand DNA is exemplified as the nucleic acid molecule, the amplification step may be appropriatly performed by selecting polymerases and primers in accordance with the subject nucleic acid molecule to be amplified, in case of using the other nucleic acid such as single strand DNA, double strand RNA and double strand RNA as the nucleic acid molecule.

So transcribed nucleic acid molecule may be as the oligonucleotide obtained in the method for obtaining oligonucleotide according to the present invention. In addition, the nucleic acid molecule may be subjected to repeating the above-mentioned steps, if necessary.

### (The other steps)

The method for obtaining oligonucleotide according to the present invention may comprise a step of using the filter in accordance with the above-mentioned method in addition to the electrophoresis step. The step of using the filter in accordance with SELEX method is the same in the prior art.

### Embodiment

### (Reference Example 1)

200 nM of oligonucleotide (sequence number 1), and 0, 200 nM, 2 µM and 4 µM of ΔNS3 protein (sequence number 2) originated from NS3 protease of hepatitis virus C wherein the His-Tag having 6 histidine residues is liked to the N terminal of the protein (hereinafter, referred simply to as ΔNS3 protein) were incubated in a binding buffer solution (20 µL of reaction volume) at 20% final concentration of glycerol (50 mM of Tris-HCl (pH7.8), 5 mM of MgCl₂ and 5 mM of CaCl₂) for 20 minutes at room temperature.

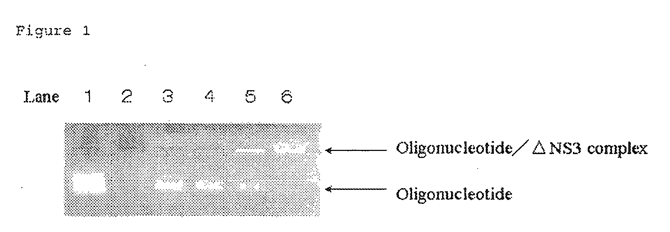

So obtained mixture was introduced in 1% of agarose gel containing 0.00005% of ethidium bromide, and an electrophoresis was performed in TBE buffer (44.5 mM Tris, 44.5 mM of boric acid and EDTA (1 mM, pH 8.0)) for 7 minutes at 100V. The gel was immersed in 1% ethidium bromide solution and was observed under UV illuminator. The result is shown in Figure 1. In Figure 1, lane 1 corresponds to tRNA, lane 2 corresponds to control, and lanes 3 to 6 correspond to mixtures containing 200 nM of oligonucleotide and ΔNS3 protein wherein the concentrations of ΔNS3 protein are 0, 200 nM, 2 µM and 4 µM, respectively.

### (Reference Example 2)

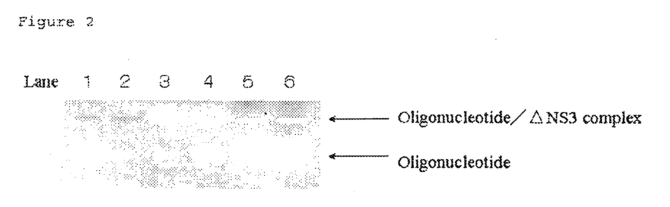

The Reference Example 2 was performed similar to the Reference Example 1, except that 2 µM of ΔNS3 protein and 0, 200 nM, 1 µM and 2 µM of tRNA (Product name: 10 109 541 001 (Roche)) were used to contain in the mixture, instead of 0, 200 nM, 2 µM and 4 µM of ΔNS3 protein, and the gel was observed. The result is shown in Figure 2. In Figure 2, lane 1 corresponds to tRNA, lane 2 corresponds to control, and lanes 3 to 6 correspond to mixtures containing 200 nM of oligonucleotide, 200 nM of ΔNS3 protein and tRNA wherein the concentrations of tRNA are 0, 200 nM, 1 µM and 2 µM, respectively.

### (Embodiment 1)

The following [Gel method] was performed for fifth generation, using 200 nM of ΔNS3 protein as the target substance. Then, the double strand DNA products at each generation were subjected to the following [Radiolabeling] to obtain radiolabeled-in vitro transcripts. The transcripts and 0 nM (no proteins), 50 nM and 100 nM of ΔNS3 protein, were subjected to the following [Binding Experiment]. The obtained radioactive intensities are shown in Table 1.

### (Embodiment 2)

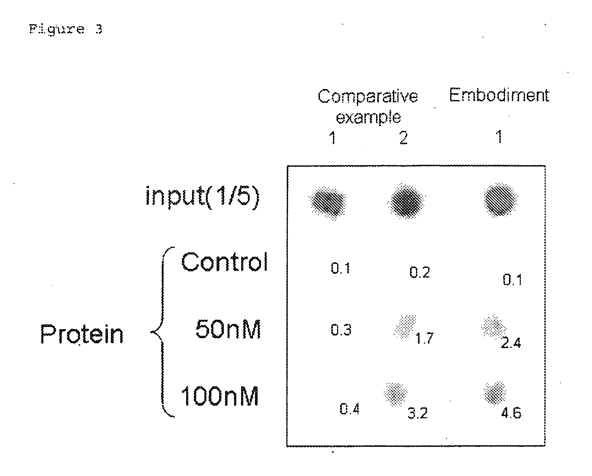

The following [Gel method] was performed for first generation, using 1 µM of mouse IgG (Catalog No. 3D3 (Hytest)) as the target substance, then the following [SELEX method] was performed for third generation. Then, the following [Radiolabeling] was performed, using the double strand DNA product obtained in the last generation, to obtain a radiolabeled in vitro transcript. The [Binding Experiment] was performed, using the transcript and 0 nM, 50 nM and 100 nM of mouse IgG, 0 nM, 50 nM and 100 nM or maltose-binding protein (MBP) (Catalog No. E8044S (New England BioLab)) and 0 nM, 50 nM and 100 nM of purified glutathicne-S-transferase (GST) (prepared in our laboratory). The obtained visualized image of the radioactive intensity is shown in Figure 3, and the values thereof are shown in Table 2.

### (Comparative Example 1)

The following [SELEX method] was performed for fifth generation, using 1 µM of mouse IgG as the target substance. Then, the following [Radiolabeling] was performed, using the double strand DNA product as obtained at the last generation, to obtain a radiolabeled in vitro transcript. The following [Binding Experiment] was performed, using the transcript and the target protein as mentioned in the Embodiment 2. The obtained visualized image of the radioactive intensity is shown in Figure 3 and the values thereof are shown in Table 2.

### (Comparative Example 2)

The Comparative Example 2 was performed as the same as the Comparative Example 1, except that the following [SELEX method] was performed for seventh generation instead of fourth generation in the Comparative Example 1. The result is shown in Figure 3 and Table 2.

### [Preparation of RNA pool]

The DNA as shown in the sequence number 3 was synthesized with DNA synthesizer (334 DNA synthesizer (Applied Biosystems)). This pool (about 10 µM) and a base sequence of a primer comprising 5'-end of T7 promoter sequence (sequence number 4) were reacted in the presence of the DNA polymerase to link the T7 promoter. Then, a transcriptional reaction was performed, using T7 RNA polymerase with so obtained double strand DNA as the template to obtain RNA pool (sequence number 5).

### [Gel method]

200 nm to 20 µM of the RNA pool was added to the binding buffer solution and stood for 5 minutes at room temperature. Then, to the solution, a metal ion (Mg2+, final concentration of 5 mM) and glycerol was added to prepare its concentration as 20%, and the target substance was added, and stood for 10 minutes at room temperature.

The mixture was introduced into wells of 1% agarose gel which is preliminary cut at the circumference of the well, and the electrophoresis was performed for 7 minutes at 100 V in TBE buffer solution.

After the electrophoresis, the solution in the well was recovered, and the oligonucleotide solution was recovered, from the agarose gel piece which is preliminary cut, with spin column (Product Name: Ultrafree-DA (Millipore)). The solution in the well and the oligonucleotide solution obtained from the agarose gel piece were subjected to ethanol precipitation to obtain an oligonucleotide.

The steps as mentioned above and the following steps from the following [RT] to the following [in vitro transcription] are correctively referred to as a first generation. In addition, each step of the next generation was performed, using the in vitro transcript as obtained in the just before generation, instead of the RNA pool.

### [SELEX method]

20 µM of the RNA pool and the target substance were mixed in the binding buffer solution, and the mixture was introduced in the nitrocellulose membrane fixed to the pop top holder to filter the mixture, and the membrane was washed with 1 mL of the binding buffer solution. Then, the membrane was immersed in 200 pL of an eluting solution (0.4 M of sodium acetate, 5 mM of EDTA and 7 M of urea (pH5.5)), and heated for 5 minutes at 90°C. So obtained solution was subjected to the ethanol precipitation to obtain an oligonucleotide.

The steps as mentioned above and the following steps from the following [RT], the following [PCR] and the following [in vitro transcription] in which the oligonucleotide is used are correctively referred to as a first generation of SELEX method. In addition, each step of the next generation was performed, using the in vitro transcript as obtained in the just before generation, instead of the RNA pool.

### [RT]

The reverse transcription reaction was performed for 1 hour at 42°C with all of the oligonucleotides was recovered, primer 1 (sequence number 6) and Reverse-iT RTase Blend (ABgene).

### [PCR]

The PCR reaction was performed for 18 cycles, using the whole volume of the reaction product of the reverse transcription reaction, 60 pg of primer 2 (sequence number 7) and primer 3 (sequence number 6) with gene Taq enzyme (Nippon Gene) to obtain a double strand DNA product, wherein one cycle of the 18 cycles corresponds to reactions for 30 seconds at 95°C, for 30 seconds at 55°C and for 30 seconds at 74°C.

### [in vitro transcription]

The in vitro transcription reaction was performed, using the double strand DNA product (0.5 µg) as obtained in the PCR reaction and T7 RNA polymerase (Product Name: Ampliscribe (EPICENTRE), volume used 1 µL) to obtain an in vitro transcript.

### [Radiolabeling]

The above-mentioned [in vitro transcription] was performed, using the above-mentioned double strand DNA product in the presence of α-³²P-ATP (Amersham Biosciences) to obtain a radiolabeled in vitro transcript.

### [Binding Experiment]

The radiolabeled in vitro transcript and the target protein were incubated in the binding buffer solution for 10 minutes at room temperature.

The obtained mixture was introduced on the filter (Product Name; MF-membrane filter (Millipore)) which is sucked with the sucker, the filter was then washed with 20 times volume of the binding buffer solution with regard to the mixture. The radioactive intensity of so obtained filter was measured with the Bioimaging analyzer (BAS-2500, FUJIFILM (using BAS-MS2040 as the imaging plate)). The radioactive intensity was visualized with ImageReader (Id.), and so obtained date was quantified with ImageGauge ver. 4.0 (Id.).

**[Table 1]**

| Generation | ΔNS3 protein | | |
|---|---|---|---|
| | 0 nM | 50 nM | 100 nM |
| Zero | 0.1 | 4.9 | 8.1 |
| First | 1.2 | 11.5 | 29.4 |
| Second | 1.4 | 16.1 | 20.8 |
| Third | 1.3 | 17.7 | 20.9 |
| Fourth | 0.5 | 9.7 | 15.4 |
| Fifth | 1.8 | 11.7 | 26.4 |

**[Table 2]**

| | Protein | | |
|---|---|---|---|
| | 0 nM | 50 nM | 100 nM |
| Embodiment 2 | 0.1 | 2.4 | 4.6 |
| Comparative Example 1 | 0.1 | 0.3 | 0.4 |
| Comparative Example 2 | 0.2 | 1.7 | 2.4 |

The values as shown in Tables 1 and 2 indicate a percentage in case that the radioactive intensity of 1/5 volume of the radiolabeled in vitro transcript before subjecting to the [Binding Experiment] is set as 20%.

The gene sequence of the in vitro transcript at the first generation in the Embodiment 1 was analyzed. As the result, 43% of these sequences were identical each other among the clone as separated and extracted. In addition, the gene sequence of the in vitro transcript at the third generation was analyzed. As the result, 66% of these sequences were identical each other among thereof.

On the other hand, the sequence identity was examined with regard to the in vitro transcript as obtained in [SELEX method], instead of [Gel method] in the Embodiment 1. As the result, the target sequences at the first generation and the third generation were not able to be detected. Both values were significantly lower than the values as obtained in the Embodiment 1.

In addition, the sequence identity of the in vitro transcript corresponding to the double strand DNA products as obtained in the Embodiment 2 was examined. As the result, it was about 66%.

### (Embodiment 2-1)

The following [Gel method 2] was performed for first generation, using 50 µM of myeloperoxidase (MPO; sequence number 9) as the target substance.

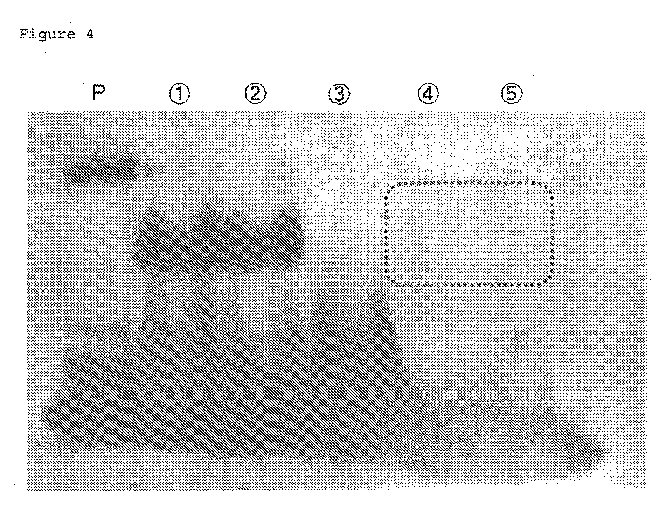

The following [PAGE method] was performed for three generations (second generation, third generation and fourth generation in addition to the previous generation), using the radiolabeled in vitro transcript at the first generation. The autoradiogram of the obtained polyacrylamide gel at the second generation, the third generation and the fourth generation are shown in Figures 4, 5 and 6, respectively.

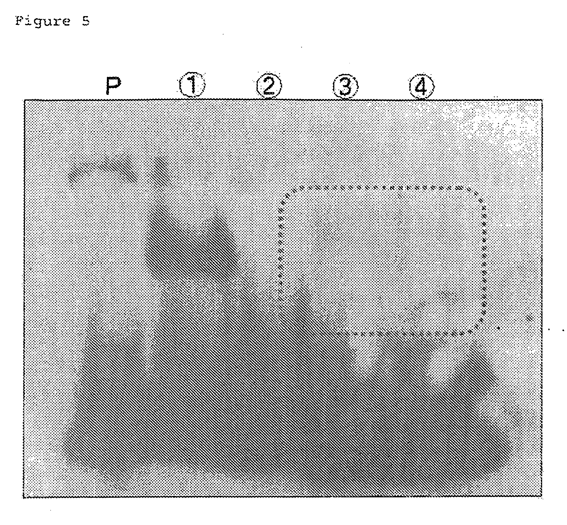

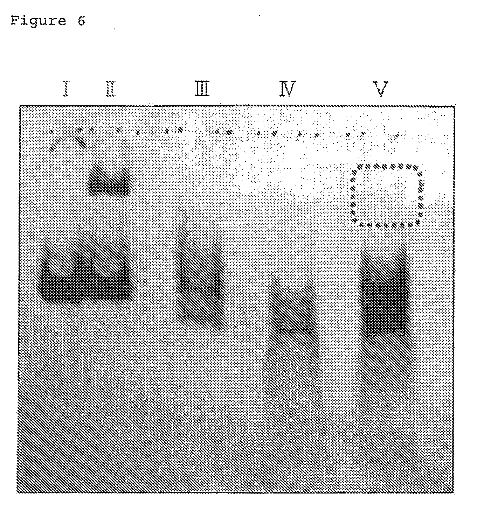

In Figure 4, lane P corresponds to the RNA pool only, lanes 1 and 2 correspond to the mouse IgG and the RNA aptamer corresponding thereto, lane 3 corresponds to yellowish-green fluorescent protein (CpYGFP; sequence number 15) only, lane 4 corresponds to MPO only, and lane 5 corresponds to the radiorabeled in vitro transcript as obtained in use of the mixture of MPO, CpYGFP and MBP, respectively. In Figure 5, lane P corresponds to the RNA pool 2 only, lanes 1 corresponds to the mouse IgG and the RNA aptamer corresponding thereto, lane 2 corresponds to CpYGFP only, lane 3 corresponds to MPO only, and lane 4 corresponds to the radiorabeled in vitro transcript as obtained in use of the mixture of MPO, CpYGFP and MBP, respectively. In Figure 6, lane I indicates the RNA aptamer to the mouse IgG, lane II indicates the mouse IgG and the RNA aptamer corresponding thereto, lane III indicates CpYGFP only, lane IV indicates the radiorabeled in vitro transcript as obtained in CpYGFP, and MPO, and lane V indicates the radiorabeled in vitro transcript as obtained in MPO, and MPO. In addition, in Figures 4 to 6, the encircled region with the dashed line indicates the gel as collected.

### [Preparation 2 of DNA pool]

The Preparation 2 of RNA pool was performed as the same as the above-mentioned [Preparation of RNA pool], except that the RNA as shown in sequence number 10 was used instead of the DNA as shown in sequence number 3, to obtain a double strand DNA. Then, a transcriptional reaction was performed, using T7 RNA polymerase with to obtained double strand DNA as the template to obtain a DNA pool 2 (sequence number 11).

### [Gel method 2]

500 pM of the RNA pool 2 was added in a binding buffer 2 (50 mM HEPES/KOH (pH 7.6), 100 mM of NaCl, 5 mM of MgCl₂ x and 20% or glycerol), and the target substance was further added, and stood for 30 minutes at room temperature.

The mixture was subjected to the above-mentioned [Gel method] to obtain an oligonucleotide.

The steps as mentioned above and the following steps from the following [RT 2], and the following [PCR 2] and the following [in vitro transcription] are correctively referred to as a first generation. In addition, each step of the next generation was performed, using the in vitro transcript as obtained in the just before generation, instead of the RNA pool 2.

### [PAGE method]

In the above-mentioned [Gel method 2], 500 pM of the RNA pool was replaced with the radiolabeled in vitro transcript at the just before generation, 1% agarose gel was replaced with polyacrylamide (8%; 45 mM of Tris, 0.45 mM of boric acid and 2.5 mM of MgCl₂), and THE buffer solution was replaced with a Running Buffer (45 mM Tris, 0.4.5 mM of boric acid, and 2.5 mM of MgCl₂) to perform the electrophoresis for 2 hours at 4°C with 160 V (CV).

While the gel was observed under the exposure with X-ray film so as to detect the radiorabeled form of the gel after the electrophoresis, a band in which the gel shift was observed was cut, and immersed in the buffer solution, RNA molecule was extracted by means of the free diffusion into the buffer solution, and was subjected to an appropriate method such as alcohol precipitation to efficiently obtain an oligonucleotide. In addition, the oligonucleotide was subjected to the [Gel method 2].

### [RT 2]

Reverse transcription reaction was performed as the same as the above-mentioned [RT], except that primer 11 (sequence number 12) was used instead of primer 1 (sequence number 6)

### [PCR 2]

The PCR 2 was performed as the same as the above-mentioned [PCR], except that primer 2 (sequence number 7) and primer 3 (sequence number 8) were replaced with primer 12 (sequence number 13) and primer 13 (sequence number 14), respectively to obtain a double strand RNA product.

### (Embodiment 2-2)

The in vitro transcript at the fourth generation as obtained in the Embodiment 2-1 was subjected to the TA cloning, and base sequences of the cloned 50 colonies were analyzed. As the result, the oligonucleotides of sequence numbers 16 and 17 were obtained.

The radiolabeled in vitro transcripts corresponding to these oligonucleotides were subjected to the above-mentioned [Binding Experiment]. Kd value and Bmax of each oligonucleotide were estimated based on the binding experiment. The result is shown in Table 3.

**[Table 3]**

| | Kd (nM) | Bmax (%) |
|---|---|---|
| Sequence Number 16 | 195 | 52 |
| Sequence Number 17 | 137.7 | 77.2 |

### (Embodiment 2-3)

The above-mentioned [Binding Experiment] was performed, using the oligonucleotide of sequence number 16 and the matter corresponding to the RNA pool 2 as the radiolabeled in vitro transcript, and using 0, 50 and 100 nM of MPO and 100 nM of bovine serum albumin (BSA) as the target protein. The result is shown in Table 4.

**[Table 4]**

| | RNA pool 2 | Sequence Number 16 |
|---|---|---|
| OnM MPO | 1.49 | 0.24 |
| 50nM MPO | 2.66 | 12.48* |
| 100nM RPO | 1.70 | 11.44* |
| BSA | 1.86 | 2.46 |

In Table 4, each value was obtained in the experiment at n=3, and mark * indicates that there is 5% or lower of significant difference with regard to the value as obtained in the RNA pool 2.

These results indicate that oligonucleotides capable of binding to the target substance can be efficiently obtained in the method for obtaining oligonucleotide according to the present invention.

The present invention has been explained in reference to the preferred embodiment of the present invention. Although the particular embodiments are illustrated to explain the present invention, it is apparent that modifications and alterations can be applied to these particular embodiments without departing from the scope of the present invention as defined in the Claims. That is, it should not be interpreted that the present invention is limited to the detail of the particular embodiment and the accompanying drawing.

## Claims

1. A method for obtaining oligonucleotide comprising the steps of:
performing an electrophoresis using an agarose gel of a nucleic acid molecule/target substance complex comprising a nucleic acid molecule and a target substance, in which the nucleic acid molecule/target substance complex has been prepared by incubation with at least one of glycerol and saccharose, whereby the amount of glycerol and/or saccharose is in the range of 10 to 30 w/v%:
recovering said nucleic acid molecule/target substance complex;
extracting the nucleic acid molecule from said nucleic acid molecule/target substance complex;
gene amplifying said nucleic acid molecule.

2. The method for obtaining oligonucleotide according to claim 1, wherein said method for obtaining oligonucleotide further comprises the step of performing the electrophoresis using a polyacrylamide gel.

3. The method for obtaining oligonucleotide according to claim 1 or 2, further comprising a step of contacting said nucleic acid molecule and said target substance, prior to the step of performing the electrophoresis.

4. The method for obtaining oligonucleotide according to any one of claims 1 to 3, wherein said oligonucleotide is RNA aptamer.

5. The method for obtaining oligonucleotide according to any one of claims 1 to 4, wherein said target substance is protein.

## Patentansprüche

1. Verfahren zum Erhalten eines Oligonukleotids, umfassend die Schritte:
Durchführen einer Elektrophorese unter Verwendung eines Agarosegels von einem Nukleinsäuremolekül/Zielsubstanz-Komplex, umfassend ein Nukleinsäuremolekül und eine Zielsubstanz, wobei der Nukleinsäuremolekül/Zielsubstanz-Komplex durch Inkubation mit wenigstens einem von Glycerol und Saccharose hergerichtet worden ist, wobei die Menge von Glycerol und/oder Saccharose in dem Bereich von 10 bis 30 w/v% ist;
Rückgewinnen des Nukleinsäuremolekül/Zielsubstanz-Komplexes;
Extrahieren des Nukleinsäuremoleküls von dem Nukleinsäuremolekül/Zielsubstanz-Komplex;
Gen-Vervielfältigen des Nukleinsäuremoleküls.

2. Verfahren zum Erhalten eines Oligonukleotids nach Anspruch 1, wobei das Verfahren zum Erhalten des Oligonukleotids ferner den Schritt des Durchführens der Elektrophorese unter Verwendung eines Polyacrylamidgels umfasst.

3. Verfahren zum Erhalten eines Oligonukleotids nach Anspruch 1 oder 2, ferner umfassend einen Schritt des Kontaktierens des Nukleinsäuremoleküls und der Zielsubstanz vor dem Schritt des Durchführens der Elektrophorese.

4. Verfahren zum Erhalten eines Oligonukleotids nach einem der Ansprüche 1 bis 3, wobei das Oligonukleotid ein RNA-Aptamer ist.

5. Verfahren zum Erhalten eines Oligonukleotids nach einem der Ansprüche 1 bis 4, wobei die Zielsubstanz ein Protein ist.

## Revendications

1. Procédé pour obtenir un oligonucléotide, comprenant les étapes consistant à ;
réaliser une électrophorèse utilisant un gel d'agarose d'un complexe molécule d'acide nucléique/substance cible comprenant une molécule d'acide nucléique et une substance cible, dans laquelle le complexe molécule d'acide nucléique/substance cible a été préparé par une incubation avec au moins un élément parmi du glycérol et du saccharose, moyennant quoi la quantité de glycérol et/ou de saccharose est comprise dans la plage de 10 à 30 % p/v ;
récupérer ledit complexe molécule d'acide nucléique/substance cible ;
extraire la molécule d'acide nucléique dudit complexe molécule d'acide nucléique/substance cible ;
réaliser une amplification génique de ladite molécule d'acide nucléique.

2. Procédé pour obtenir un oligonucléotide selon la revendication 1, où ledit procédé pour obtenir un oligonucléotide comprend en outre l'étape consistant à réaliser l'électrophorèse en utilisant un gel de polyacrylamide.

3. Procédé pour obtenir un oligonucleotide selon la revendication 1 ou 2, comprenant en outre une étape consistant à mettre en contact ladite molécule d'acide nucléique et ladite substance cible, avant l'étape de réalisation de l'électrophorèse.

4. Procédé pour obtenir un oligonucléotide selon l'une quelconque des revendications 1 à 3, dans lequel ledit oligonucléotide est un aptamère d'ARN.

5. Procédé pour obtenir un oligonucleotide selon l'une quelconque des revendications 1 à 4, dans lequel ladite substance cible est une protéine.


### Sequence Listing

<110> NEC Soft, Ltd.
   National Institute of Advance Industrial Science And Technology
<120> Method for obtaining nucleotides
<130> 101-04712
<150> JP2006-212046
   <151> 2006-08-03
<160> 17
<170> Patent In version 3.4
<220> 1
   <211> 74
   <212> DNA
   <213> Artificial
<220>
   <223> G9-II (10/23)
<400> 1
<210> 2
   <211> 179
   <212> PRT
   <213> Escherichia coli
<400> 2
   Pro Ile Thr Ala Tyr Ser Gln Gln Thr Arg Gly Leu Leu Gly Cys Ile
   1 5 10 15
   Ile Thr Ser Leu Thr Gly Arg Asp Lys Asn Gln Val Glu Gly Glu Val
   20 25 30
   Gln Val Val Ser Thr Ala Thr Gln Ser Phe Leu Ala Thr Cys Ile Asn
   35 40 45
   Gly Val Cys Trp Thr Val Tyr His Gly Ala Gly Ser Lys Thr Leu Ala
   50 55 60
   Gly Pro Lys Gly Pro Ile Thr Gln Met Tyr Thr Asn Val Asp Gln Asp
   65 70 75 80
   Leu Val Gly Trp Pro Ala Pro Pro Gly Ala Arg Ser Met Thr Pro Cys
   85 90 95
   Thr Cys Gly Ser Ser Asp Leu Tyr Leu Val Thr Arg His Ala Asp Val
   100 105 110
   Ile Pro Val Arg Arg Arg Gly Asp Ser Arg Gly Ser Leu Leu Ser Pro
   115 120 125
   Arg Pro Ile Ser Tyr Leu Lys Gly Ser Ser Gly Gly Pro Leu Leu Cys
   130 135 140
   Pro Ser Gly His Val Val Gly Ile Phe Arg Ala Ala Val Cys Thr Arg
   145 150 155 160
   Gly Val Ala Lys Ala Val Asp Phe Val Pro Val Glu Ser Met Glu Thr
   165 170 175
   Thr Met Arg
<210> 3
   <211> 97
   <212> DNA
   <213> Artificial
<220>
   <223> Primary pool for preparation of RNA pool
<220>
   <221> misc_feature
   <222> (22)..(76)
   <223> n is a, c, g, or t
<400> 3
   ccctccacct tgacttcctc tnnnnnnnnn nnnnnnnnnn nnnnnnnnnn nnnnnnnnnn
   60
   nnnnnnnnnn nnnnnntgaa gcgttagcga gatgcgt
   97
<210> 4
   <211> 34
   <212> DNA
   <213> Artificial
<220>
   <223> Promotor for T7RNA polymerase
<400> 4
   tgtaatacga ctcactatag gtagatacga tgga 34
<210> 5
   <211> 97
   <212> RNA
   <213> Artificial
<220>
   <223> RNA pool
<220>
   <221> misc_feature
   <222> (22) .. (76)
   <223> n is a, c, g, or t
<400> 5
   gggaggugga acugaaggag annnnnnnnn nnnnnnnnnn nnnnnnnnnn nnnnnnnnnn
   60
   nnnnnnnnnn nnnnnnacuu cgcaaucgcu cuacgca
   97
<210> 6
   <211> 21
   <212> DNA
   <213> Artificial
<220>
   <223> primer for RT-PCR
<400> 6
   tgcgtagagc gattgcgaag t 21
<210> 7
   <211> 55
   <212> DNA
   <213> Artificial
<220>
   <223> primer for PCR (5')
<400> 7
   tgtaatacga ctcactatag gtagatacga tggagggagg tggaactgaa ggaga
   55
<210> 8
   <211> 21
   <212> DNA
   <213> Artificial
<220>
   <223> primer for PCR (3')
<400> 8
   acttcgcaat cgctctacgc a 21
<210> 9
   <211> 745
   <212> PRT
   <213> Homo sapiens
<400> 9
<210> 10
   <211> 79
   <212> DNA
   <213> Artificial
<220>
   <223> none
<220>
   <221> misc_feature
   <223> n is a, c, g, or t
<220>
   <221> misc_feature
   <222> (35)..(64)
   <223> n is a, c, g, or t
<400> 10
   agtaatacga ctcactatag gtagatacga tggannnnnn nnnnnnnnnn nnnnnnnnnn 60
   nnnncatgac gcgcagcca 79
<210> 11
   <211> 79
   <212> RNA
   <213> Artificial
<220>
   <223> none
<220>
   <221> misc_feature
   <223> n is a, c, g, or t
<220>
   <221> misc_feature
   <222> (35) .. (64)
   <223> n is a, c, g, or u
<400> 11
   aguaauacga cucacuauag guagauacga uggannnnnn nnnnnnnnnn nnnnnnnnnn
   60
   nnnncaugac gcgcagcca 79
<210> 12
   <211> 15
   <212> DNA
   <213> Artificial
<220>
   <223> none
<400> 12
   tggctgcgcg tcatg 15
<210> 13
   <211> 34
   <212> DNA
   <213> Artificial
<220>
   <223> none
<400> 13
   agtaatacaa ctcactatag gtagatacga tgga 34
<210> 14
   <211> 15
   <212> DNA
   <213> Artificial
<220>
   <223> none
<400> 14
   catgacgcgc agcca 15
<210> 15
   <211> 213
   <212> PRT
   <213> Escherichia coli
<400> 15
<210> 16
   <211> 60
   <212> RNA
   <213> Escherichia coli
<400> 16
   gguagauacg auggaacacg aagauuucaa agugauaccc cagggcauga cgcgcagcca
   60
<210> 17
   <211> 60
   <212> RNA
   <213> Escherichia coli
<400> 17
   gguagauacg auggaacacg aauauuucaa agugauaccc cagggcauga cgcgcagcca 60